# EUROPEAN PATENT APPLICATION

(11) **EP 2 865 337 A1**
(43) Date of publication of application: **29.04.2015**
(21) Application number: 13189896.7
(22) Date of filing: 23.10.2013
(51) Int. Cl.: A61B 7/02

(54) **Stethoscope head cover**

(71) Applicant: Malchers, Florian, 51427 Bergisch Gladbach (DE)
(72) Inventor: Malchers, Florian, 50679 Cologne (DE); Fischer, Christian, 51105 Cologne (DE); Wecker, Stefan, 50935 Cologne (DE)
(74) Representative: Vossius & Partner Patentanwälte Rechtsanwälte mbB

(57) **Abstract**

Stethoscope head cover (10) for covering a patient contacting site of a stethoscope head, the cover comprising: a ring-shaped mounting portion (11) for coupling the cover to an outer circumferential rim of the stethoscope head (1), the mounting portion comprising a membrane receiving portion (13) for attaching a membrane (15) thereto; a shield (17) extending from the mounting portion that is configured to overlap a part (5,9) of the stethoscope head which is spaced from the patient contacting site; and holding means (12) for holding the stethoscope head cover to the stethoscope head.

## Description

The present invention relates to stethoscope head covers for protecting the head of a stethoscope from contact with a patient and, in particular, to disposable, single-use stethoscope head covers comprising a ring shaped mounting for receiving a membrane.

Stethoscopes are well-known in the art and are frequently used by physicians and other medical practitioners for auscultation. These will be referred to as "users" in the following specification.

Acoustic stethoscopes usually comprise a head or chest piece and hollow tubes for transmitting the sound from the chest piece to the ears of the user. The stethoscope head may comprise two opposing sides, a bell section and a diaphragm section with a diaphragm, which may be placed against the skin of a patient for identifying body sounds of lower and higher frequencies, respectively. The diaphragm section of known stethoscope heads may comprise a diaphragm and a diaphragm retaining ring. The diaphragm retaining ring may be latched or screwed onto the head in order to affix the diaphragm and taut it. While the term "bell section" will be used for the side of the head lacking a diaphragm in accordance with the usual terminology in the art for the purposes of the following description, it is noted that such diaphragm-less sections or sides of the head may have any suitable shape and the invention is applicable to any of these shapes. As such, the bell section may or may not have the shape of a bell.

Furthermore, also one-sided stethoscope heads featuring a single auscultation section (with or without a diaphragm) are known in the art. The present invention is equally applicable to these types of stethoscope heads.

Since the stethoscope head is placed against the patient (e.g. in direct contact with the patient's skin) when listening to body sounds, users must disinfect and/or sterilize the head after each use. This may be a time consuming and expensive procedure for physicians and medical practitioners that see numerous patients each day. Moreover, germs may be transmitted from one patient to another in the event of inadequate cleaning.

In order to reduce the time spent on disinfection between patients and the risk of cross-infections, different types of covers for stethoscope heads have been developed. While, for example, US Patent No. 5,921,941 to Longobardo et al., US Patent No. 4,461,368 to Plourde, WO 2005/030054 to Agahi, US Patent No. 5,365,023 to Lawton, and US Patent No. 5,564,431 to Seward, which are all incorporated herein by reference in their entirety, disclose different types of covers, to date, known covers have not been widely accepted by the medical community for different reasons, including difficulty in use, lack of sterility, and dampening of body sounds. Accordingly, there is a persisting need for further improvements in the field.

In view of the above, it is an object of the present invention to provide improved stethoscope head covers as well as associated devices and methods in order to overcome some or all drawbacks of the prior art. In particular, it is an object of the present invention to provide stethoscope head covers that are easily applied, that minimize the risk of cross-infections, and/or that have a low dampening effect.

The above-mentioned objects are achieved by stethoscope head covers according to the claims. Further aspects, improvements and variations are disclosed in the Figures and in the description. Some or all of these aspects, improvements and variations may be combined in a single embodiment of the invention. Moreover, some of these aspects may also form distinct inventions on their own and may be claimed as such.

### Mounting portion

According to an aspect, the present invention relates to a stethoscope head cover for covering a patient contacting site of a stethoscope head, i.e. the part of the head that is placed against the skin of a patient during auscultation. The cover comprises a ring-shaped mounting portion for coupling it to known stethoscope heads and, more specifically, for coupling it to an outer circumferential rim of a bell section and/or a diaphragm section of such head. In this context, the outer circumferential rim of the diaphragm section may be an outer circumferential rim of a diaphragm retaining ring or an outer circumferential rim of the head on which the diaphragm retaining ring is mounted. In the latter case, the diaphragm retaining ring and the diaphragm may be removed before attaching the inventive cover to the head.

The mounting portion may comprise holding means for holding the stethoscope head cover to the stethoscope head, which holding means may be formed as one, several or continuous protrusions (e.g., one or several wedge-shaped protrusions) extending away from the ring-shaped mounting portion towards the centre of the ring. Alternatively or additionally, other means for providing a positive lock or form fit may be provided, for example, a screw thread on the inner side of the mounting portion.

In line with the teachings of the present invention, the mounting portion may be made from a relatively stiff material, for example, a relatively stiff polymeric material. This may allow the mounting portion to be self-supporting and maintain its shape when detached from the head, which may facilitate attachment of the cover. For example, the mounting portion may be formed of a material having an elastic modulus (E-modulus) of at least 0.1 GPa or at least 0.5 GPa. Alternatively or additionally, the E-modulus may be 10 GPa or less or 7 GPa or less. Further in line with the present invention, the mounting portion may be formed of a material having a shore hardness of about 30 to about 70, preferably of about 50 to about 70 on a type A scale (i.e., preferably measured with a 35° truncated cone that has a front surface diameter of 0.79 mm, e.g., according to DIN ISO 7619-1).

The mounting portion may form a continuous ring that extends along 360° and/or have a substantially cylindrical cross-section. In order to further facilitate attachment of the cover to the head, the inner diameter of the mounting portion may substantially correspond to the outer diameter of the outer circumferential rim of the bell section and/or of the diaphragm section of the stethoscope head. For example, the inner diameter of the mounting portion may essentially correspond to the outer diameter of the rim or may be less than 2% or less than 5% smaller than said outer diameter.

### Membrane receiving portion

The mounting portion may further comprise a membrane receiving portion for attaching a membrane thereto, which membrane may be used instead of or in addition to a membrane integrated into the diaphragm section of the stethoscope head. As such, a twofold membrane or double-layer structure, which results when a regular stethoscope head cover is placed over a diaphragm integrated into the diaphragm section, may be avoided and dampening may be reduced. Particular membranes may be provided to the inventive cover for different stethoscope types and/or different auscultation purposes in order to improve the acoustic performance.

The membrane receiving portion of the stethoscope cover may be configured to support the membrane on the side of the cover that contacts the patient such that the membrane is positioned between the patient's skin and the stethoscope head. In order to provide optimal acoustical performance and minimize dampening, the membrane receiving portion may be configured to firmly clamp the membrane against the stethoscope head in use. Moreover, acoustical performance may be enhanced by configuring the membrane receiving portion such that the membrane is taut when the cover is latched onto the stethoscope head.

The membrane receiving portion may provide a circumferential membrane attachment surface that is essentially ring-shaped and extends from the mounting portion towards the centre of the ring. Accordingly, the membrane attachment surface may extend transversal to the mounting portion in an inward direction and the inner diameter of the membrane attachment surface may be smaller than the inner diameter of the mounting portion. In this context, the mounting portion and the attachment portion together may form an L-shaped, J-shaped or stepped cross-section in a plane parallel to the longitudinal axis of the mounting portion (i.e., the axis through the centre of the ring). The membrane attachment surface may be planar, convex, or concave.

The membrane receiving portion may be relatively stiff and self-supporting such that it maintains its shape when the cover is detached from the stethoscope head. For example, the membrane receiving portion may be formed of a material having an E-modulus of at least 0.1 GPa or at least 0.5 GPa. Alternatively or additionally, the E-modulus may be 10 GPa or less or 7 GPa or less. Also, the membrane receiving portion may be formed of a material having a shore hardness of about 30 to about 70, preferably of about 50 to about 70 on a type A scale. The membrane receiving portion may be integrally formed with the mounting portion.

### Membrane

The stethoscope head cover of the present invention may be manufactured, delivered and/or used with or without a membrane. While a membrane may be particularly advantageous when the cover is employed with a diaphragm section, the membrane may omitted, for example, when the cover is intended to be use with a stethoscope head or the side of a stethoscope head that normally does not feature a diaphragm (e.g., the bell section of a two-sided stethoscope head). In the latter case, the mounting portion and/or the membrane receiving portion may cover the surfaces of the stethoscope head, thereby avoiding contact between the head and the patient in order to provide adequate protection.

When a membrane is used, said membrane may be formed of the same materials, shaped like and/or have the same or similar acoustical characteristics than regular stethoscope membranes used for commonplace stethoscope heads. According to embodiments of the invention, a specific membrane may be provided in accordance with the stethoscope head for which the cover is intended. This membrane may have the same or similar characteristics than the diaphragm of the respective head.

While the membrane may be integrally formed with the membrane receiving portion in some embodiments of the invention, it is currently preferred to provide the membrane as a separate element. In this case, the membrane may be fixed to the attachment surface by any suitable method, for example, welding and/or gluing.

### Shield

In order to reduce the risk of cross-infections further, the stethoscope head of the present invention may comprise a shield that covers a part of the stethoscope head which is not in direct contact with the patient during use, in particular, a part of the head section employed (e.g., the bell section or the diaphragm section) that does not come into contact with the skin of the patient during auscultation. For example, the part covered by the shield may be a part of the stethoscope head that is placed under a cuff when measuring blood pressure with a sphygmomanometer. Thus, cross-infections via the cuff me be prevented.

The shield preferably extends from the end of the mounting portion that faces away from the mounting portion's patient contacting side, i.e., from the end of the mounting portion that is opposite the membrane receiving portion. More specifically, the shield may extend from the mounting portion in a cantilevered manner, for example, such that an angle ***α*** measured on the inner side of the cover when considering a cross-sectional plane parallel to the longitudinal axis is larger than 90°, preferably between 110° and 150°.

In line with the teachings of the present invention, the shield may be made from a relatively stiff material, e.g., a relatively stiff polymeric material. This may allow the shield to be self-supporting and maintain its shape when detached from the head, which may further facilitate attachment of the cover to the head. For example, the shield may be formed of a material having an E-modulus of at least 0.1 GPa or at least 0.5 GPa. Alternatively or additionally, the E-modulus may be 10 GPa or less or 7 GPa or less. Further in line with the present invention, the shield may be formed of a material having a shore hardness of about 30 to about 70, preferably of about 50 to about 70 on a type A scale. The shield may be integrally formed with the mounting portion and/or from the same material than said mounting portion.

In order to avoid interference of the shield with the stethoscope head during attachment of the cover, the shield may extend only along a part of the mounting portion and not along the entire circumference thereof. For example, the shield may extend along the circumference of the mounting portion with an arc of the circumference being spared, which arc may have a central angle *γ* of at least 90°, at least 120°, at least 150° or at least 180°. The shield may extend along an arc having a central angle ***β*** of at least 100°, at least 120°, at least 180°, at least 270°.

The mounting portion, the membrane attachment portion and/or the shield may be formed from a material featuring low heat conduction (e.g. a polymeric material) in order to avoid giving the patient an unpleasant cold feeling when the cover touches the skin.

### Dispenser and Projections on the stethoscope head cover

The stethoscope head of the present invention may be provided in a dispenser, which dispenser may also form an invention on its own and be claimed as such.

The inventive dispenser preferably presents a single stethoscope head cover to the user and allows the user to latch this cover onto a stethoscope head without requiring the user to touch and/or grasp the cover. For this purpose, the dispenser may comprise a reservoir chamber for receiving a stack of covers and a push mechanism for pushing said stack towards a single-release unit that successively releases the covers. For example, the dispenser may be configured to present the uppermost cover of the stack to the user and then push the entire stack upwards once the uppermost cover is removed.

The dispenser and/or the stethoscope cover of the present invention may be provided with rotation-prevention means in order to avoid rotation of the cover in the dispenser, in particular, in order to avoid rotation of the cover within the single-release unit. By preventing rotation, latching of the cover onto the head may be facilitated. Also, misalignment of the cover, which might prompt the user to touch it, may be avoided.

The rotation-prevention means may be provided, for example, by one or several projections of the cover (e.g., two projections), which preferably extend away from the outer periphery of the mounting portion in an outward direction. Each of these one or several projections may mate with a corresponding slot in the dispenser. However, as will be apparent to those skilled in the art, the one or several projections may also be provided in the dispenser, in which case corresponding slots may be formed in the mounting portion of the cover. Moreover, also other rotation-prevention means may be used. For example, the outer periphery of the mounting portion may be shaped in a non-circular or irregular manner and a corresponding aperture may be provided in the reservoir chamber of the dispenser.

### Protective sheet

Further according to the present invention, the membrane may be covered by a protective sheet that can be peeled off before use. The protective sheet may be provided with one or several tabs that can be grasped by a user, which tabs may extend past the outer circumference of the mounting portion. Furthermore, the protective sheet may have a different colour than the membrane in order to provide the user with a visible feedback indicating that the respective cover has not been used yet.

### Weakness area

The stethoscope head cover may further comprise a weakness area in order to facilitate removal and avoid repeated use. Preferably, the weakness area is formed along the mounting portion and/or the shield of the cover and may be provided, for example, by a row of perforations and/or a section with reduced wall thickness. The stethoscope head cover may be a disposable devise intended for single-use.

### Standardising ring

As a further component, the stethoscope head cover according to the present invention may comprise one or several standardising rings for adapting the outer diameter of the outer circumferential rim of the respective stethoscope head or stethoscope head section to the inner diameter of the mounting portion. Such standardising rings may allow using the same cover size for the heads of numerous stethoscopes that are currently on the market and thereby substantially increase the flexibility of the cover according to the present invention. The standardising ring may be configured to be fitted onto the outer rim of a diaphragm section or the outer rim of a bell section. Alternatively, the standardising ring may be configured to be fitted onto the diaphragm section instead of the diaphragm retaining ring.

The standardising ring may comprise one or several projections, bayonet fittings, and/or a screw threadings for attaching the standardising ring to the stethoscope head and/or the stethoscope head cover. The projections, bayonet fitting, and/or screw threading may be provided on the inner and/or the outer side of the standardising ring.

When a standardising ring is used, the inner diameter of the mounting portion may substantially correspond to the outer diameter of the standardising ring or may be less than 2% or less than 5% smaller than said outer diameter.

### Method

According to a further aspect, the present invention also relates to a method for attaching a stethoscope head cover to a diaphragm section or a bell section of a stethoscope head. A stethoscope head cover according to the present invention may be used in this context.

When the stethoscope head cover is attached to a diaphragm section, the inventive method may comprise the step of attaching the cover to the diaphragm section after removing the membrane or diaphragm that is incorporated into said diaphragm section (e.g., the membrane incorporated into the diaphragm section when the stethoscope head is delivered to the user). Then, the stethoscope head cover of the present invention may be attached to the diaphragm section and the membrane of the stethoscope head cover may be used as a the diaphragm, reducing the overall damping effect considerably. For example, the stethoscope head cover may replace the diaphragm retaining ring after the standard diaphragm is removed. Alternatively, the diaphragm retaining ring may be fixed to the diaphragm section again (without putting the standard diaphragm back into place) and the stethoscope head cover may be attached to said diaphragm retaining ring. The stethoscope head cover according to the present invention, therefore, may be used on a diaphragm section of a stethoscope without a diaphragm being provided in addition to the membrane of the cover.

According to further aspects, the method may also comprise a step of attaching a standardising ring to an outer circumferential rim of the diaphragm section (e.g. an outer circumferential rim exposed after removing the diaphragm retaining ring or an outer circumferential rim of said diaphragm retaining ring). In this case, the stethoscope head cover of the present invention may be attached to the standardising ring.

The aspects of the invention addressed above will also become fully apparent form the following Figures, which show embodiments of the invention for illustrational purposes. The Figures are schematic drawings only and embodiments shown may be modified in many ways within the scope of the claims. In particular, the disclosure provided by the Figures is not meant to limit the scope of protection conferred by the invention. The Figures show:
- Fig. 1A: a schematic side view of a stethoscope head;
- Fig. 1B: a schematic top view of the stethoscope head of Fig. 1A;
- Fig. 1C: a schematic side view of a stethoscope head provided with a standardising ring in accordance with the present invention;
- Fig. 2A: a schematic perspective view of a stethoscope head cover in accordance with the present invention;
- Fig. 2B: a schematic top view of the stethoscope head cover of Fig. 2A;
- Figs. 3A-3C: schematic cross sections of stethoscope heads in accordance with the present invention;
- Figs. 4A-4C: a schematic sequence of top views illustrating the mounting of a stethoscope head cover in accordance with the invention onto a stethoscope head;
- Figs. 5A-5C: side views of the sequence shown in Figs. 4A-4C;
- Fig. 6: a schematic side view of a dispenser for stethoscope head covers in accordance with the present invention.

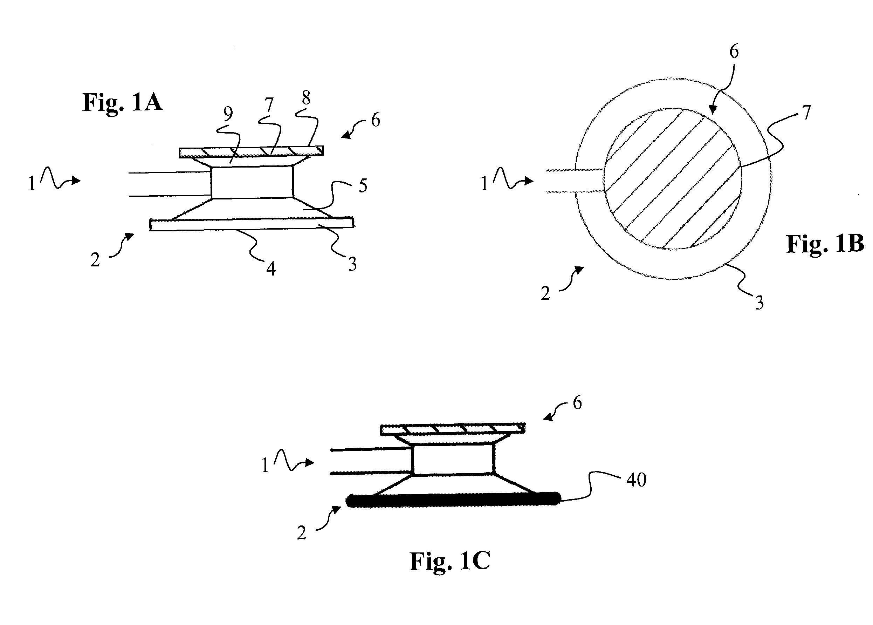

Figs. 1A and 1B show schematic side and top views, respectively, of a conventional stethoscope head 1 comprising a diaphragm section 2 and a bell section 6. The diaphragm section 2 has an outer rim 3, a patient contacting side 4 and a part 5 which is not in direct contact with the patient during auscultation and is normally placed under a cuff when measuring blood pressure with a sphygmomanometer. Similarly, the bell section 6 has an outer rim 7, a patient contacting side 8 and a part 9 which is not in direct contact with the patient during auscultation. Fig. 1C shows the stethoscope head of Figs. 1A and 1B with the diaphragm retaining ring and the diaphragm of the diaphragm section 2 being removed and a replaced by a standardising ring 40 according to the present invention. Alternatively, the standardising ring 40 may also be provided over the outer rim 3 or the outer rim 7.

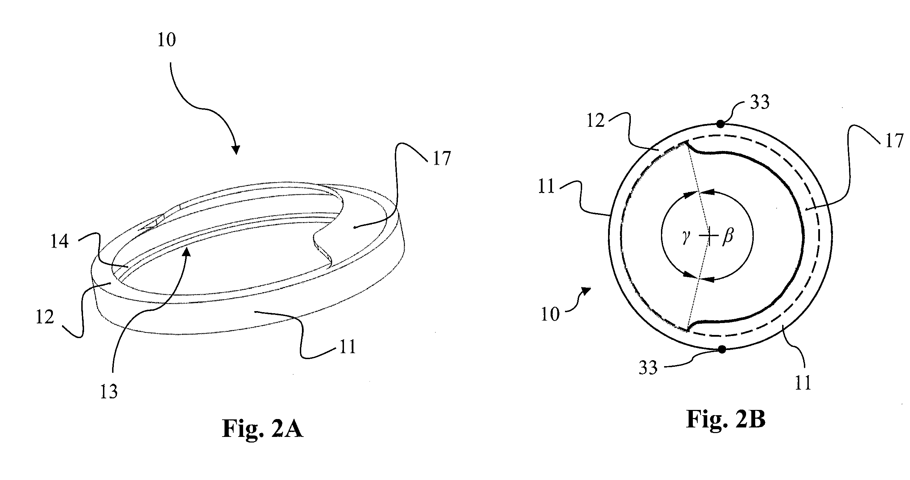

Fig. 2A shows an exemplary embodiment of a stethoscope head cover 10 in accordance with the present invention in a perspective view, which may either be attached directly to the outer rim 3 of the diaphragm section 2 or the outer rim 7 of the bell section 6 of the head 1 shown in Figs. 1A and 1B or by means of the standardising ring 40 shown in Fig. 1C.

As further shown in Fig. 2A, the cover 10 comprises a ring-shaped mounting portion 11 with a membrane receiving portion 13 formed at the end of the mounting portion 11 that faces towards patient during auscultation. The membrane receiving portion 13 provides a circumferential surface 14 for attaching a membrane (not shown in Fig. 2A). The surface 14 may at least extend along sections of the circumference of the mounting portion 11 and preferably extends along the entire circumference of said mounting portion 11.

On the opposite side of the mounting portion 11, the exemplary stethoscope head cover 10 illustrated in Fig. 2A comprises a self-supporting shield 17 that projects from the mounting portion 11 towards the centre of the cover 10. As also apparent from the schematic top view in Fig. 2B, the shield 17 does not extend along the entire circumference of the mounting portion 11. Rather, an arc having a central angle *γ* of 120° to 160° is spared in the exemplary embodiment and the shield 17 merely extends along the remainder of the circumference of the mounting portion 11, i.e., along an arc having a central angle *β* of 200° to 240°. In embodiments, a central angle *γ* of 120° to 180° may be spared and the shield 17 may merely extend along the remainder of the circumference of the mounting portion 11, i.e., along an arc having a central angle ***β*** of 180° to 240°.

The mounting portion 11 may also be provided with one or several protrusions 12 for providing a stronger attachment of the cover 10 to the head. As shown in Fig. 2A, these protrusions 12 may be formed as a continuous protrusion along at least a segment of the mounting portion 11 or the entire mounting portion 11. The protrusion 12 may be located, e.g., along the inner circumferential surface of the mounting portion 11.

As further schematically shown in Fig. 2B, the cover 10 may be provided with projections 33 for preventing rotation of the cover 10 in a dispenser. This may be, for example, two projections or pins extending outwards from the mounting portion 11 at opposite sides of the cover 10.

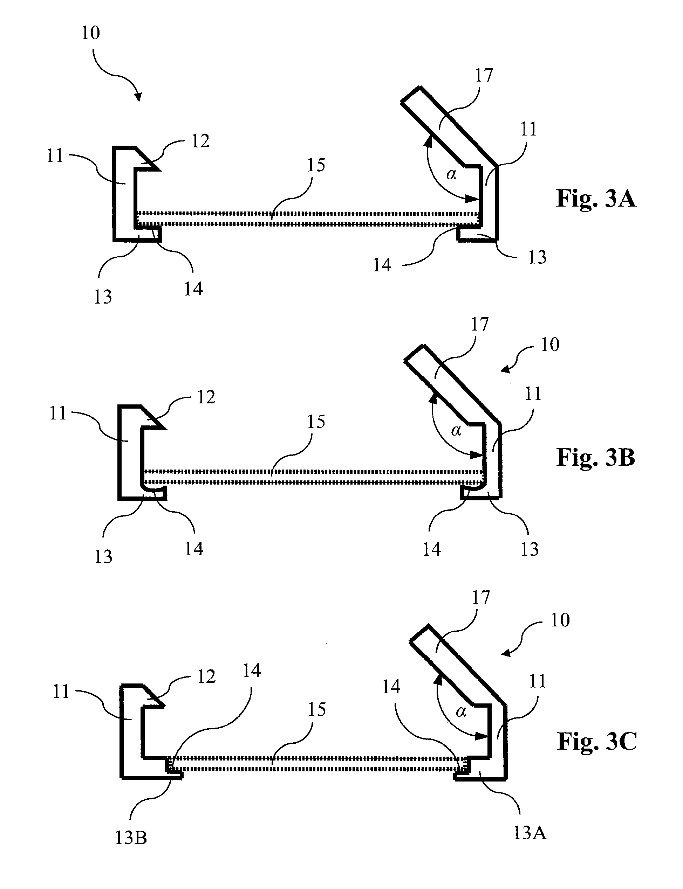

Turning to the schematic cross-sections of covers 10 according to the present invention that are illustrated in Figs. 3A-3C, it is noted that the mounting portion 11 may be provided with holding means for holding the cover 10 to a stethoscope head. To facilitate attachment of the cover 10 to the head, these holding means may be formed, for example, by one or several protrusions 12 that project from an inner surface of the mounting portion 11, which may be continuous along at least a segment of the mounting portion or the entire mounting portion. As shown, an upper portion of the protrusions 12 may be wedge-shaped in order to allow for easy insertion of the head into the cover 10. In embodiments, also a round or hemispherical protrusion 12 may be employed.

With continued reference to the cross-sections of Figs. 3A-3C, it will be appreciated that the shield 17 may project from the mounting portion 11 at an angle ***α*** measured between the mounting portion 11 and the shield 17 on the inner side of the cover 10. In the exemplary embodiments illustrated in the Figures, the angle ***α*** may be between 110° and 150°, for example. As shown, the mounting portion 11, the membrane receiving portion 13, the shield 17 and/or the protrusions 33 may be integrally formed from the same material.

As further shown in Figs. 3A-3C, the stethoscope cover 10 of the present invention may be provided with a membrane 15. More specifically, the membrane 15 may be attached and/or fixed to the mounting portion 11 at the patient contacting end thereof by means of the membrane receiving portion 13 such that the membrane 15 is disposed between the receiving portion 13 and the stethoscope head when the cover 10 is latched onto such head.

Depending on the materials employed and the particular application, the membrane attachment surface 14 of the membrane receiving portion 13 may be provided with different shapes, as, for example, a planar shape (see Fig. 3A), a concave shape (see Fig. 3B), or a convex shape (not shown). Therefore, the mounting portion 11 and the membrane receiving portion 13 together may form the L-shaped or J-shaped cross-section shown in Figs. 3A and 3B, respectively. Alternatively or additionally, the membrane receiving portion 13 may have a stepped configuration (see Fig. 3C) and comprise a thicker outer part 13A and a thinner inner part 13B providing the surface 14 to which the membrane 15 is attached. In any case, the membrane 15 may be attached to the circumferential surface 14 by any suitable means such as, for example, welding or the use of adhesives.

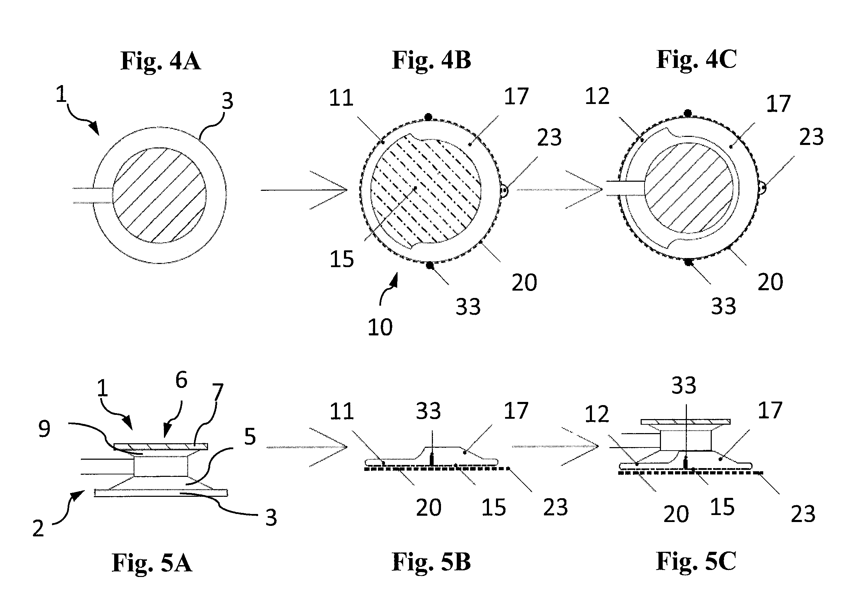

Figs. 4A-4C and 5A-5C show a sequence of top and side views, respectively, that illustrate the mounting of a stethoscope head cover 10 in accordance with the present invention onto a stethoscope head 1. As shown, the head 1 of Fig. 4A is introduced into the cover 10 of Fig. 4B from the side of the cover 10 that is exempt of the shield 17. The outer rim 3 of the diaphragm section 2 may then be engaged with the section of the mounting portion 11 covered by the shield 17. Subsequently, the stethoscope head 1 may be pressed downwards, such that also the remaining part of the mounting portion 11 receives the outer rim 3 and the protrusion 12 latches onto the stethoscope head 1.

As further schematically shown in Figs. 4B, 4C, 5B and 5C, a protective sheet 20 (which may be coloured) covers the membrane 15 and indicates to the user that the cover 10 has not been employed yet. The protective sheet 20 may be removed before placing the stethoscope head 1 with the cover 10 against the skin of the patient.

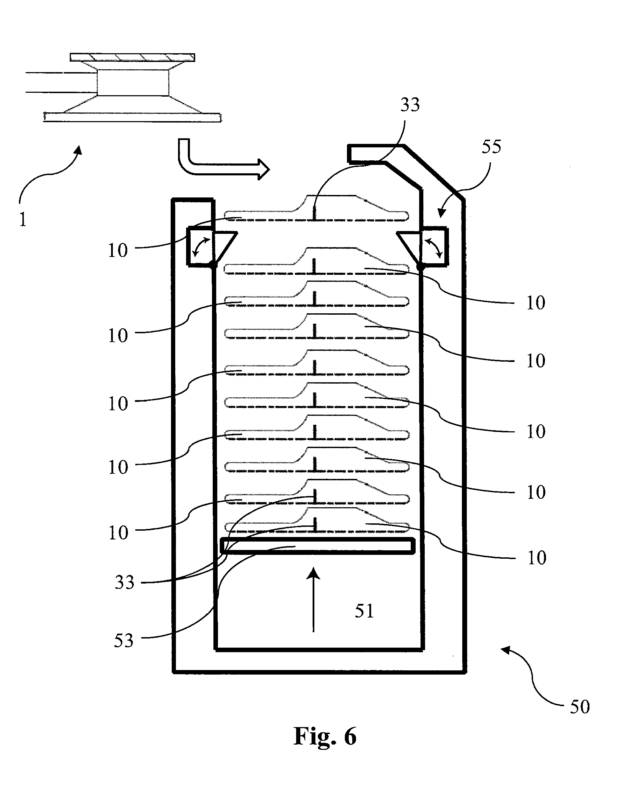

Finally, Fig. 6 shows a schematic side view of a dispenser 50 for stethoscope head covers 10 in accordance with the present invention. The dispenser 50 has a reservoir chamber 51 in which a stack of covers 10 may be received. A push mechanism 53 pushes the stack of covers 10 towards a single-release unit 55 that is provided at an end of the reservoir chamber 51. The single-release unit 55 presents a single cover 10 of the stack in the reservoir to the user, allowing the user to directly engage said cover 10 with a stethoscope head 1, without having to touch, grasp, or manipulate the cover 10 with the hands. Therefore, the cover can be easily applied and contamination is avoided.

When the uppermost cover 10 is removed, the single-release unit 55 dispenses the next cover 10 of the stack and presents it to the user. The user of the stethoscope may then tear the cover 10 off the head 1 and latch the next cover 10 provided by the dispenser 50 onto the head 1.

In view of the above, the present invention provides several advantages over prior art stethoscope covers. In particular, aspects and embodiments of the invention provide covers that may be easily attached and detached from a stethoscope head, while still providing a reliable and tight fit. Since the membrane of the cover may be used as the diaphragm, dampening is also reduced. Moreover, the inventive dispenser and protective sheet substantially reduce the risk of contamination before use, even if the user is not wearing gloves.

While the invention has been illustrated and described in detail in the drawings and the foregoing description, such illustration and description are to be considered illustrative or exemplary and not restrictive. It will be understood that changes and modifications may be made by those of ordinary skill within the scope of the following claims. In particular, the present invention covers further embodiments with any combination of features from different embodiments described above. As far as the words "substantially" or "generally" are used, the present disclosure also encompasses embodiments in which the respective feature/s is/are entirely fulfilled.

## Claims

1. Stethoscope head cover (10) for covering a patient contacting site of a stethoscope head (1), the cover comprising:
a ring-shaped mounting portion (11) for coupling the cover (10) to an outer circumferential rim (3, 7) of the stethoscope head (1), the mounting portion (11) comprising a membrane receiving portion (13) for attaching a membrane (15) thereto,
a shield (17) extending from the mounting portion (11) and being configured to overlap a part (5, 9) of the stethoscope head (1) that is spaced from the patient contacting site; and
holding means (12) for holding the stethoscope head cover (10) to the stethoscope head (1).

2. Stethoscope head cover (10) according to claim 1, wherein the holding means (12) are provided by one, several or continuous protrusions extending from the mounting portion (11).

3. Stethoscope head cover according to claim 1 or 2, wherein the membrane receiving portion (13) is essentially ring-shaped and smaller in diameter than the mounting portion (11), and wherein the membrane receiving portion (13) comprises a membrane attachment surface (14) configured to support the membrane between the membrane receiving portion (13) and the stethoscope head (1) such that the membrane (15) is clamped against the stethoscope head (1) in use.

4. Stethoscope head cover (10) according to claim 3, wherein membrane attachment surface (14) is planar or concave and/or wherein the membrane receiving portion (13) has a stepped configuration.

5. Stethoscope head cover (10) according to any of the preceding claims, wherein the mounting portion (11) has an essentially cylindrical form, the mounting portion (11) being self-supporting when the cover (10) is detached from the stethoscope head (1).

6. Stethoscope head cover (10) according to any of the preceding claims, wherein the mounting portion (11), the shield (17) and/or the attachment portion (13) is made from a polymeric material that has an E-modulus of 0.1 GPa to 10 GPa, preferably of 0.5 GPa to 7 GPa.

7. Stethoscope head cover (10) according to any of the preceding claims, wherein the mounting portion (11), the shield (17) and/or the attachment portion (13) is made from a polymeric material that has a shore hardness of about 30 to about 70, preferably of about 50 to about 70 on the type A scale.

8. Stethoscope head cover (10) according to any of the preceding claims, wherein the shield (17) projects from the mounting portion (11) in a cantilevered manner, the shield being self-supporting.

9. Stethoscope head cover (10) according claim 8, wherein an angle (**α**) measured in a cross-section of the stethoscope head cover (10) on the inner side of said cover (10) between the mounting portion (11) and the shield (17) is larger than 90° and preferably between 110° and 150°.

10. Stethoscope head cover (10) according to any of the preceding claims, wherein the shield (17) extends along the circumference of the mounting portion (11), an arc of the mounting portion's circumference having a central angle (***γ***) of at least 90°, at least 120°, at least 150°, or at least 180° being spared.

11. Stethoscope head cover (10) according to any of the preceding claims, wherein the shield (17) extends along an arc having a central angle (***β***) of at least 100°, at least 120°, at least 180°, or at least 270°.

12. Stethoscope head cover (10) according to any of the preceding claims, wherein the mounting portion (11) comprises at least one projection (33) configured to prevent rotation of the cover (10) in a dispenser (50), preferably wherein the projection (33) extends away from the outer periphery of the mounting portion (11).

13. Stethoscope head cover (10) according to any of the preceding claims, further comprising one or several standardising rings (40) in order to match the diameter of the outer circumferential rim (3, 7) to the inner diameter of the mounting portion (11).

14. Method for attaching a stethoscope head cover (10) according to any of the preceding claims to a diaphragm section (2) of a stethoscope head (1), the method comprising the steps of
- removing the diaphragm retaining ring and the diaphragm from the stethoscope head (1); and
- attaching the cover according any of the preceding claims onto the diaphragm section (2).

15. Method according to claim 14, the method further comprising the step of attaching a standardising ring (40) to an outer circumferential rim (4) of the diaphragm section (2), the cover (10) being subsequently attached to said standardising ring (40).
